# EUROPÄISCHE PATENTSCHRIFT

(11) **EP 3 730 101 B1**
(45) Veröffentlichungstag und Bekanntmachung des Hinweises auf die Patenterteilung: **21.08.2024**
(21) Anmeldenummer: 19171379.1
(22) Anmeldetag: 26.04.2019
(51) Int. Cl.: A61F 9/00, B65D 51/16, B65D 47/18

(54) **BELÜFTETE SCHUTZKAPPE FÜR EINEN FLÜSSIGKEITSSPENDER**
VENTED PROTECTIVE CAP FOR A LIQUID DISPENSER
CAPUCHON DE PROTECTION VENTILÉ POUR UN DISTRIBUTEUR DE LIQUIDE

(43) Veröffentlichungstag der Anmeldung: 28.10.2020
(73) Patentinhaber: Aptar Radolfzell GmbH, 78315 Radolfzell (DE)
(72) Erfinder: Greiner-Perth, Jürgen, 78244 Gottmadingen (DE); Herz, Andi, 78253 Eigeltingen-Reute (DE)
(74) Vertreter: Witte, Weller und Partner Patentanwälte mbB Stuttgart

(56) Entgegenhaltungen:
- DE-B- 1 218 305
- DE-B3- 102016 210 992
- DE-B4- 102013 226 253
- US-B1- 6 174 576

## Beschreibung

Die Erfindung betrifft eine belüftete Schutzkappe, einen Belüftungseinsatz insbesondere für eine solche Schutzkappe sowie einen Flüssigkeitsspender mit einer solchen Schutzkappe. Die Erfindung betrifft weiterhin auch korrespondiere Herstellungsverfahren.

Belüftete Schutzkappen finden bei Flüssigkeitsspendern für pharmazeutische Flüssigkeiten Verwendung, um nach Erstgebrauch einen Luftaustausch zwischen einem Kappeninnenraum und einer Umgebung zu gestatten. Dies dient dem Zweck des schnelleren Abtrocknens von Flüssigkeitsresten, die jenseits der Austragöffnung eines Spenders verblieben sind. Hierdurch wird Bakterienwachstum unterbunden. Die Belüftungsöffnungen von belüfteten Schutzkappen führen ihrerseits jedoch auch zur Gefahr eines Keimeintrags aus der Umgebung, so dass bereits vorgeschlagen wurde, die Belüftungsöffnung mit einem Sterilfilter zu versehen.

Aus der DE 102013226253 B4 ist es bekannt, einen solchen Sterilfilter an der Kappe vorzusehen. Dabei schlägt das genannte Dokument auch bereits vor, einen zweilagigen Einsatz zu verwenden, der aus einer Trägerschicht besteht, auf der der Sterilfilter fest angebracht ist.

Die Verwendung einer solchen mehrlagigen Struktur ist von Vorteil, um mehrere Funktionen erfüllen zu können. Allerdings ist die Herstellung eines mehrlagigen flexiblen Flächenmaterials vergleichsweise aufwändig, so dass sich derartiges nur für große Serien lohnt bzw. die Herstellungskosten deutlich erhöht.

Aus Dokument US 6,174,576 B1 ist es bekannt, Fiberwolle zwischen Lagen aus Polypropylen zu platzieren, wobei die Lagen aus Polypropylen verschweißt werden, um mit der Fieberwolle befüllte Belüftungskanäle für Behälter zu schaffen.

Aufgabe der Erfindung ist es, eine belüftete Schutzkappe bzw. einen Belüftungseinsatz insbesondere für eine solche Schutzkappe zur Verfügung zu stellen, die preisgünstig die Anpassung an spezifische Anforderungen gestattet.

Zur Lösung dieser Aufgabe wird eine belüftete Schutzkappe für einen Flüssigkeitsspender vorgeschlagen, die eine Kappenwandung aufweist, die einen Kappeninnenraum umgibt. Die Kappenwandung weist eine Belüftungsdurchbrechung auf, die von einer flexiblen Flächenstruktur überspannt wird. Ein Randbereich der Flächenstruktur ist umlaufend an einem Rand der Belüftungsdurchbrechung fixiert.

Die Flächenstruktur einer erfindungsgemäßen Schutzkappe besteht aus mindestens zwei Lagen, die unmittelbar aneinander anliegen, jedoch zumindest in einem Zentralbereich der Flächenstruktur nicht fest miteinander verbunden sind.

Die Flächenstruktur überspannt die mindestens eine Belüftungsdurchbrechung und gewährleistet so, dass ein Luftaustausch zwischen einem Kappeninnenraum und einer Umgebung bei aufgesetzter Schutzkappe nur durch die Flächenstruktur hindurch möglich ist. Die Flächenstruktur weist eine Mehrzahl von Lagen auf, die vorzugsweise unterschiedliche Funktionen erfüllen. Diese Lagen werden bei der Herstellung getrennt gehandhabt und sind zumindest in einem Zentralbereich von vorzugsweise mindestens 9 mm² nicht unmittelbar miteinander verbunden. In einem Randbereich können sie im Zuge der Herstellung jedoch miteinander verbunden werden, insbesondere durch gemeinsames Umspritzen mit Kunststoff oder thermische Fügung aneinander.

Durch die mindestens zwei Lagen, die nicht gemeinsam aus einem Verbundmaterial stammen, sondern jeweils einzeln aus Flächen oder Bahnen ihres spezifischen Typs erzeugt werden, ist ein hohes Maß an Flexibilität gegeben. Die mehreren Lagen können auch für kleine Serien von Spendern anforderungsspezifisch zusammengestellt werden.

Exemplarisch seien die folgenden Lagen genannt, die Teil einer Flächenstruktur der erfindungsgemäßen Spenderkappe sein können.

Üblicherweise umfasst ist eine Lage, die als Sterilfilter ausgebildet ist. Dieser weist vorzugsweise eine Trenngrenze von maximal 1 µm auf, insbesondere vorzugsweise eine Trenngrenze von maximal 0,5 µm. Ein noch bessere Sterilität lässt sich mit einer Trenngrenze von maximal 0,2 µm erzielen. Ein solcher Sterilfilter verhindert den Eintritt von Keimen durch die Belüftungsdurchbrechung.

Die Filterlage kann als Tiefenfilter oder als Membranfilter ausgebildet sein. Der Tiefenfilter weist eine dreidimensionale Struktur auf, in die Bakterien zwar eintreten können, die jedoch die Bakterien aus der durchströmenden Luft abscheidet. Beim Membranfilter sind Poren definierter Größe vorgesehen, mittels derer Keime bereits vor Eintritt in die Lage abgeschieden werden.

Eine Lage der Flächenstruktur kann als saugfähige Lage ausgebildet sein, die zur Aufnahme eines Resttropfens von einer Austragöffnung des Spenders vorgesehen ist, wobei diese Lage ausgehend vom Kappeninnenraum vorzugsweise die erste Lage der Flächenstruktur darstellt. Diese saugfähige Lage kann bei aufgesetzter Schutzkappe unmittelbar an der Stirnfläche der Haupteinheit des Spenders anliegen oder von dieser Stirnfläche gering beabstandet sein.

Die Lagen können eine hydrophile Oberfläche oder eine hydrophobe Oberfläche aufweisen. So kann es beispielsweise sinnvoll sein, eine saugfähige Lage hydrophil auszugestalten, um die Aufnahme von Flüssigkeit zu beschleunigen. Auch können mehrere Lagen in unterschiedlichem Maße hydrophil ausgestaltet sein, um Restflüssigkeit hierdurch aufzunehmen und zu einer besonders hydrophilen Kernlage zu leiten.

Auch kann es sinnvoll sein, eine Lage vorzusehen, die antibakteriell ausgebildet ist oder einen bakterientötenden Inhaltsstoff aufweist. Hierdurch können in der Luft oder in aufgenommener Flüssigkeit enthaltene Bakterien beim Durchtritt durch die Lagen abgetötet werden und zumindest deren Wachstum verringert werden.

Auch kann eine Lage der Flächenstruktur als Stützlage ausgebildet sein, insbesondere eine äußerste Lage. Eine solche Stützlage kann der Flächenstruktur mechanische Stabilität verleihen. Hierdurch können weitere Lagen der Flächenstruktur gegen versehentliche und mutwillige Verletzungen geschützt sein.

Insbesondere kann es sich um eine Art festes Gewebe handeln. Auch eine Gestaltung mit mehreren Stützlagen, insbesondere beidseitig einer Sterilfilterlage, kann zweckmäßig sein.

Eine weitere fallweise zweckmäßige Lage ist eine Sichtschutzlage, die mindestens eine darunterliegende Lage kaschiert. Dies ist insbesondere bei Schutzkappen sinnvoll, die farbig gestaltet sind. Da Sterilfilter meist eine technisch bedingte Farbgebung aufweisen und häufig weiß sind, fallen diese an farbigen Schutzkappen unmittelbar auf und verursachen ggf. das Missverständnis, die entsprechende Lage sei bestimmungsgemäß zu entfernen. Eine Sichtschutzlage in Farbgebung des Kunststoffs der Schutzkappe versteckt den Sterilfilter und beugt daher dem genannten Missverständnis vor.

Bei einer ersten Variante einer erfindungsgemäßen Schutzkappe ist vorgesehen, dass die mindestens zwei Lagen der Flächenstruktur durch eine gemeinsame angespritzte Tragstruktur am Rand der Belüftungsdurchbrechung gehalten sind. Dabei ist vorgesehen, dass die Tragstruktur einseitig oder insbesondere vorzugsweise einstückig beidseitig der Flächenstruktur über den Randbereich der Flächenstruktur hinüberragt.

Insbesondere das beidseitige Halten wird als vorteilhaft angesehen. Hierbei ragen zwei umlaufende Stege der Tragstruktur oberhalb und unterhalb der flexiblen Flächenstruktur über diese hinüber und fixieren die Flächenstruktur dadurch. Die Herstellung erfolgt vorzugsweise dadurch, dass zunächst die mehrlagige Flächenstruktur in eine Spritzgusskavität eingebracht wird und dann von Kunststoff umspritzt wird, der dabei die genannten beiden umlaufenden Stege bildet.

Aber auch Gestaltungen mit nur einem solchen Steg können sinnvoll sein. Die diesem Steg abgewandten Lagen sind dann umfänglich durch Kunststoffmaterial gehalten. Insbesondere bei porösen Lagen wie einem Tiefenfilter lässt sich trotz der nur geringen Fläche am Umfang eine feste Verbindung mit dem Kunststoff der Kappe schaffen.

Bei einer anderen Variante einer erfindungsgemäßen Schutzkappe ist vorgesehen, dass am Rand der Belüftungsdurchbrechung eine Tragstruktur mit einer ringförmigen Befestigungsfläche vorgesehen ist. Eine erste Lage der Flächenstruktur ist in einem Randbereich thermisch an dieser Befestigungsfläche befestigt. Eine zweite Lage der Flächenstruktur ist in einem Randbereich thermisch zumindest auch am Randbereich der ersten Lage befestigt.

Bei dieser Art der Gestaltung sind die Lagen der Flächenstruktur nicht umspritzt, sondern thermisch an der genannten Befestigungsfläche bzw. aneinander befestigt. Die Verbindung ist dabei vorzugsweise mittels eines hohlen Prägestempels erzeugt, der im Randbereich die jeweiligen Lagen komprimiert und ausschmilzt, so dass sie miteinander bzw. mit der Befestigungsfläche eine innige Verbindung eingehen. Auch andere Fügetechniken wie Laserschweißen und Ultraschallschweißen sind hier möglich.

Die Schutzkappe kann ein Hauptbauteil aufweisen, welches mindestens zum überwiegenden Teil eine Mantelwandung der Kappenwandung bildet und an dem die Tragstruktur einstückig vorgesehen ist. In einem solchen Falle kann die Kappe mit Ausnahme der Lagen der Flächenstruktur über der Belüftungsdurchbrechung somit einstückig hergestellt sein.

Neben dieser Gestaltung mit einstückig an der der Mantelwandung vorgesehener Tragstruktur kann auch vorgesehen sein, dass die Schutzkappe ein Hauptbauteil aufweist, welches mindestens zum überwiegenden Teil eine Mantelwandung der Kappenwandung bildet und eine Öffnung zur Aufnahme eines Belüftungseinsatzes aufweist. Die Schutzkappe weist in diesem Fall als weitere Komponente einen in die Öffnung dichtend eingesetzten Belüftungseinsatz auf, der die umgebende Tragstruktur und die hiervon gehaltene und umgebene Flächenstruktur umfasst.

Eine solche modulare Bauweise geht zwar mit etwas höherem Herstellungsaufwand einher. Sie gestattet es jedoch, einheitliche Hauptbauteile zu verwenden und diese durch Verwendung eines geeigneten Belüftungseinsatzes anforderungsspezifisch anzupassen.

Bei einer einfachen Bauweise einer erfindungsgemäßen Schutzkappe ist vorgesehen, dass nur eine Belüftungsdurchbrechung vorgesehen ist, die durch die Tragstruktur oder hiermit einstückig verbundene Abschnitte nicht segmentiert wird. Insbesondere in solchen Fällen kann es von Vorteil sein, wenn die Schutzkappe eine Schutzstruktur zum Schutz der Flächenstruktur aufweist, wobei die Schutzstruktur als von der Tragstruktur getrenntes Bauteil ausgebildet ist und mit der Tragstruktur kraftschlüssig oder formschlüssig verbunden ist. Diese Schutzstruktur, die vorzugsweise als separates Kunststoffteil hergestellt ist, kann in noch sichererem Maße als die oben genannte Stützlage die Flächenstruktur schützen.

Die Erfindung betrifft neben der Kappe auch den bereits beschriebenen Belüftungseinsatz, insbesondere zur Verwendung in einer erfindungsgemäßen Kappe.

Auch hier ist vorgesehen, dass der Belüftungseinsatz eine Belüftungsdurchbrechung aufweist, die von einer Tragstruktur umgeben ist und dass die Belüftungsdurchbrechung von einer flexiblen Flächenstruktur überspannt ist, deren Randbereich umlaufend an der Tragstruktur fixiert ist.

Die Flächenstruktur besteht dabei aus den bereits beschriebenen mindestens zwei Lagen, die unmittelbar aneinander anliegen, jedoch zumindest in einem Zentralbereich der Flächenstruktur nicht fest miteinander verbunden sind. Die hier möglichen Lagen umfassen alle oben in Hinblick auf die Schutzkappe genannten Lagen.

Wie auch bei der beschriebenen Schutzkappe, kann auch bei einem entsprechenden Belüftungseinsatz vorgesehen sein, dass die zwei Lagen der Flächenstruktur durch eine gemeinsame angespritzte Tragstruktur am Rand der Belüftungsdurchbrechung gehalten sind, die einseitig oder einstückig beidseitig der Flächenstruktur über den Randbereich der Flächenstruktur hinüberragt. Alternativ kann auch hier vorgesehen sein, dass am Rand der Belüftungsdurchbrechung eine Tragstruktur mit einer ringförmigen Befestigungsfläche vorgesehen ist, wobei eine erste Lage der Flächenstruktur in einem Randbereich thermisch mit der Befestigungsfläche verbunden ist und wobei eine zweite Lage der Flächenstruktur in einem Randbereich thermisch mit dem Randbereich der ersten Lage verbunden ist.

Die Verbindung des Belüftungseinsatzes kann in einer Aufnahme, insbesondere der beschriebenen Schutzkappe, mittels unterschiedlicher Verbindungstechniken befestigt sein. Eine bevorzugte Gestaltung sieht vor, dass der Belüftungseinsatz eine sich verjüngende und vorzugsweise zumindest abschnittsweise konische Mantelfläche aufweist, die ein leichtes Einsetzen und ggf. das Herstellen einer selbsthemmenden Presspassung mit der Aufnahme gestattet.

Alternativen dazu sehen vor, dass der Belüftungseinsatz mittels Schnappverbindung befestigt wird und dementsprechend eine hinterschnappungsfähige Außengeometrie aufweist. Auch eine Gewindeverbindung oder eine Kleb- oder Schweißverbindung sind möglich. Im Falle einer Schweißverbindung kann diese insbesondere mittels Laserschweißen oder Ultraschallschweißen erzeugt werden.

Die Erfindung betrifft neben der beschriebenen Schutzkappe und dem beschriebenen Belüftungseinsatz auch einen Flüssigkeitsspender, insbesondere für pharmazeutische Flüssigkeiten. Dieser Flüssigkeitsspender verfügt über eine Haupteinheit, die ihrerseits einen Flüssigkeitsspeicher, eine Fördereinrichtung und eine Austragöffnung zur Abgabe der Flüssigkeit umfasst. Die Fördereinrichtung kann insbesondere als Pumpeinrichtung zur Förderung von drucklos gelagerter Flüssigkeit oder als Ventileinrichtung zur Förderung von druckbeaufschlagt in einem Druckspeicher gelagerter Flüssigkeit ausgebildet sein. Bei manueller Betätigung der Fördereinrichtung gelangt Flüssigkeit vom Flüssigkeitsspeicher zur Austragöffnung. Statt einer vom Flüssigkeitsspeicher getrennten Fördereinrichtung kann der Flüssigkeitsspeicher auch mit einer Quetschflasche versehen sein, also eine durch manuelle Kraftbeaufschlagung komprimierbaren Flasche, die somit selbst die Fördereinrichtung darstellt.

Insbesondere kann der Flüssigkeitsspender als Tropfenspender ausgebildet sein, also zur Abgabe von Einzeltropfen, insbesondere für die Applikation in die Nase, in die Ohren oder in die Augen. Ein solcher Tropfenspender weist vorzugsweise die Austragöffnung umgebend eine Tropfenbildungsgeometrie auf, beispielweise in Form einer konkaven oder ebenen Tropfenbildungsfläche, die vorzugsweise durch eine scharfkantige Abrisskante umgeben ist. Insbesondere zum Austrag von konservierungsmittelfreien Flüssigkeiten ist es von Vorteil, wenn der Austragöffnung ein Abgabeventil vorgeschaltet ist, welches hier den Keimeintrag verhindert, gleichzeitig aber auch die Rücksaugung eines Resttropfens unterbindet.

Insbesondere zum Zwecke des schnellen Trocknens eines solchen Resttropfens weist der Flüssigkeitsspender erfindungsgemäß eine belüftete Schutzkappe oben beschriebener Art auf, die auf die Haupteinheit aufsetzbar ist und die Austragöffnung im aufgesetzten Zustand schützt. An dieser Schutzkappe oder bei einer alternativen Verwendung an anderer Stelle eines Flüssigkeitsspenders kann erfindungsgemäß ein Belüftungseinsatz oben beschriebener Art vorgesehen sein.

Die Verwendung einer erfindungsgemäßen Schutzkappe und/oder einer erfindungsgemäßen Belüftungseinheit kann auch bei anderen Spendern, beispielsweise auch bei Sprühspendern zum zerstäubten Austrag von Flüssigkeit zweckmäßig sein.

Die Schutzkappe ist dabei vorzugsweise mit einer Flächenstruktur mit einer saugfähigen oder antibakteriell ausgestalteten Lage versehen, wobei diese Lage bei aufgesetzter Schutzkappe unmittelbar oberhalb der Austragöffnung angeordnet ist, so dass ein an der Austragöffnung verbliebener Resttropfen hiervon aufgenommen und/oder dekontaminiert werden kann.

Der Flüssigkeitsspender ist im Lieferzustand mit einer pharmazeutischen Flüssigkeit befüllt. Insbesondere handelt es sich um pharmazeutische Flüssigkeiten zur Behandlung des erhöhten Augeninnendrucks (Glaukombehandlung), zur Behandlung des trockenen Auges und zur Behandlung von Allergien und Entzündungen. Hierbei spielen insbesondere die Molekülgruppen Alpha-2 Agonisten, z. B. Brimonidin, Prostaglandinanaloga (Tafluprost, Latanoprost, Bimatoprost, Travoprost), Beta-Blocker, z. B. Timolol, und Carboanhydrasehemmer, z. B. Dorzolamid beziehungsweise Hyaluronsäureverbindungen, Filmbildner, z. B. Methylcelluloseverbindungen und Cyclosporin beziehungsweise Antihistaminika, z. B. Olopatadin und Levocabastin, Steroide, z. B. Loteprdnol und Dexamethason, sowie NSAID, z. B. Keterolac, eine Rolle.

Weiterhin ist der erfindungsgemäße Spender vorteilhaft verwendbar für Flüssigkeiten mit Molekülen von einer oder von mehreren der folgenden Sorten: Trichloressigsäure, Trioxysalen, Harnstoff, Zinkoxid, Tacrolimus, Clobetasolpropionat, Mometasonfuroat, Betamethasondipropionat, Fluocinonid, Desoximetasone, Triamcinolon Acetonid, Fluticasonpropionat, Hydrokortison, Clotrimazol, Ketoconazol, Miconazol, Undecylensäure, Terbinafine, Cyclopirox, Tolnaftate, Akziklovir, Imiquimod, Docosanol, Finasterid, Minoxidil, Dexamethason, Tramazolin, Naphazolin, Nostrilla, Oxymethazolin, Phenylephrin, Phenylpropanolamin, Pseudoephedrin, Tetryzolin, Tramazolinhydrochlorid, Tuaminoheptane und Xylometazolin.

Die Erfindung betrifft darüber hinaus auch Verfahren zur Herstellung einer Schutzkappe oben beschriebener Art oder eines Belüftungseinsatzes oben beschriebenerArt. Im Weiteren wird dies primär anhand einer Schutzkappe erläutert, wobei die Verfahrensschritte entsprechend auch zur Herstellung eines separaten Belüftungseinsatzes Verwendung finden können, der dann anschließend insbesondere in die Aufnahme einer Schutzkappe eingesetzt wird.

Gemäß einer ersten Variante der Erfindung werden eine erste Lage eines ersten flächigen Materials und eine zweite Lage eines zweiten flächigen Materials kontinuierlich in Form flächiger Bänder zugeführt. Sofern mehr Lagen Verwendung finden sollen, sind entsprechend weitere flächige Bänder vorzusehen.

In einem überlappenden Bereich der Bänder werden die mindestens zwei Lagen durch einen oberseitigen Stempel und einen unterseitigen Stempel zusammengedrückt und es wird die Stempel umgebend mit einer Schneidkontur ein Schnitt vorgenommen, mit dem die zwei Lagen der für die Schutzkappe oder den Belüftungseinsatz vorgesehenen Flächenstruktur von den flächigen Bändern getrennt wird. Zu diesem Zeitpunkt werden die beiden miteinander nicht fest verbundenen Lagen zwischen den beiden Stempeln gehalten.

Diese mehrlagige Flächenstruktur wird anschließend in durch die Stempel fixiertem Zustand in eine Spritzgusskavität eingefügt. In die Spritzgusskavität wird dann ein Kunststoffmaterial, beispielsweise ein HDPE oder PP, eingespritzt, welches eine gemeinsame Tragstruktur bildet, die einseitig oder einstückig beidseitig der Flächenstruktur über den Randbereich der Flächenstruktur hinüberragt und die Lagen der Flächenstruktur fixiert.

Die beidseitigen Stempel werden nach Aushärtung der Tragstruktur von der Flächenstruktur beabstandet und geben hierbei die Pfade von einer äußeren Umgebung bis zur Flächenstruktur und von der Flächenstruktur in einen Innenbereich der Schutzkappe frei.

Die Besonderheit bei dem beschriebenen Verfahren ist insbesondere, dass die gleichen Mittel, nämlich die beiden genannten Stempel, verwendet werden, um die mindestens zwei miteinander zu diesem Zeitpunkt nicht verbundenen Lagen aneinanderzupressen und sie derart in der Kavität zu positionieren, dass sie beim Spritzguss an der gewünschten Stelle gehalten werden und dort von Kunststoff umgeben werden. Die Stempel selbst halten dabei die Belüftungsdurchbrechung beidseitig der Flächenstruktur von Kunststoff frei.

Bei einem alternativen Verfahren ist es vorgesehen, dass zunächst ein Grundkörper einer belüfteten Schutzkappe oder eines Belüftungseinsatz zur Verfügung gestellt wird, der über eine Belüftungsdurchbrechung verfügt, die von einer Tragstruktur mit einer ringförmigen Befestigungsfläche umgeben ist.

In der Belüftungsdurchbrechung werden nacheinander oder gemeinsam eine erste Lage der Flächenstruktur und eine zweite Lage der Flächenstruktur derart platziert, dass der Randbereich der ersten Lage in Kontakt mit der ringförmigen Befestigungsfläche gelangt und dass der Randbereich der zweiten Lage in Kontakt mit dem Randbereich der ersten Lage gelangt.

Zur Befestigung der Lagen aneinander bzw. an der ringförmigen Befestigungsfläche wird mittels eines erhitzten Stempels nach Einfügung der zweiten Lage eine thermische Fügung der Randbereiche der ersten Lage und zweiten Lage untereinander bewirkt.

Die Verwendung des erhitzten Stempels kann einmalig nach Einbringung der zweiten Lage erfolgen. Hierbei erfolgt dann mittelbar auch die Befestigung der ersten Lage am ringförmigen Befestigungsbereich. Alternativ kann der erhitzte Stempel jedoch auch mehrfach, insbesondere je Lage einmal, verwendet werden. Es wird somit bereits nach der Platzierung der ersten Lage und vor Platzierung der zweiten Lage mittels des erhitzten Stempels der Randbereich der ersten Lage thermisch mit der ringförmigen Befestigungsfläche verbunden.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 und 2 zeigen einen erfindungsgemäßen Spender mit einer belüfteten Schutzkappe, die über eine Belüftungsdurchbrechung verfügt, welche mittels einer flexiblen Flächenstruktur überdeckt ist.
Fig. 3A bis 3C zeigen drei Varianten belüfteter Schutzkappen in geschnittener Darstellung.
Fig. 4A bis 4D verdeutlichen ergänzend zu den Gestaltungen der Fig. 3A und 3C die Möglichkeit der direkten Verbindung des Hauptkörper der Schutzkappe mit der flexiblen Flächenstruktur sowie der Verwendung eines separaten Belüftungseinsatzes.
Fig. 5A bis 5D zeigen verschiedene Konfigurationen von Lagen der flexiblen Flächenstruktur.
Fig. 6 und 7A bis 7H verdeutlichen ein erstes Verfahren zur Herstellung einer erfindungsgemäßen Schutzkappe.
Fig. 8A bis 8D sowie 9A bis 9F verdeutlichen zwei Variationen eines alternativen Verfahrens zur Herstellung einer erfindungsgemäßen Schutzkappe.

### DETAILLIERTE BESCHREIBUNG DER AUSFÜHRUNGSBEISPIELE

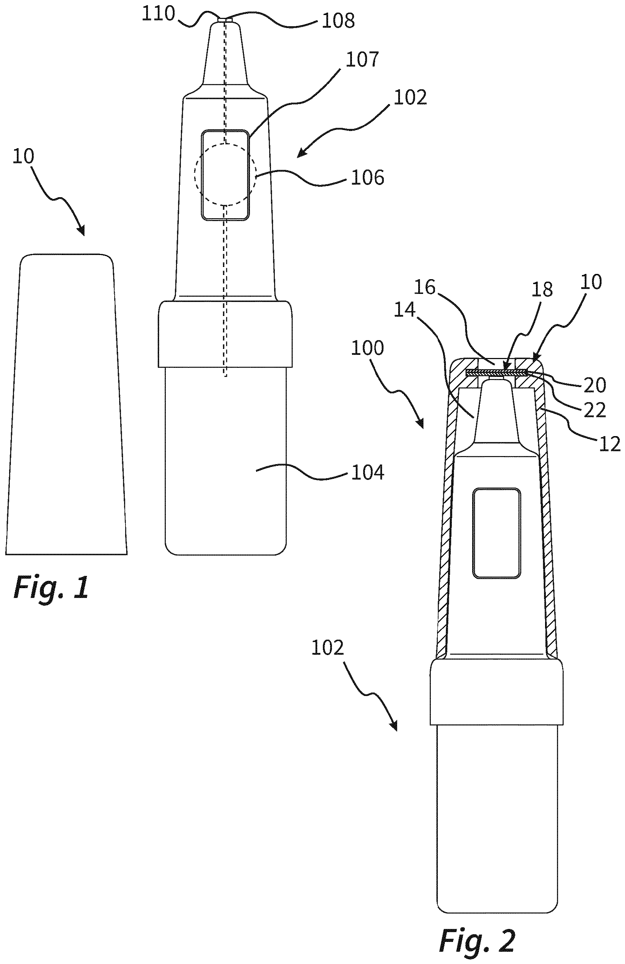

Die Fig. 1 und 2 zeigen einen erfindungsgemäßen Flüssigkeitsspender 100.

Dieser umfasst eine Haupteinheit 102, in der ein Flüssigkeitsspeicher 104, eine Fördereinrichtung 106 sowie eine Austragöffnung 108 vorgesehen sind. Die Fördereinrichtung 106 ist dafür vorgesehen, Flüssigkeit aus dem Flüssigkeitsspeicher 104 bis zur Austragöffnung 108 zu leiten. Hierbei sind verschiedene technische Gestaltungen denkbar, insbesondere die einer Gestaltung des Spenders mit einem Druckspeicher 104 und einer als Schaltventil ausgebildeten Fördereinrichtung 106. Sobald ein Benutzer auf einen Betätigungsknopf 107 drückt, wird das Schaltventil geöffnet und die unter Druck im Flüssigkeitsspeicher 104 stehende Flüssigkeit strömt zur Austragöffnung 108. Hierzu alternativ kann die Fördereinrichtung 106 als Pumpeinrichtung 106 ausgebildet sein. In jedem Fall ist die Flüssigkeit im Flüssigkeitsspeicher 104 drucklos gelagert und wird durch Betätigen der Pumpeinrichtung 106 mittels des Betätigungsknopfs 107 zur Austragöffnung 108 gepumpt. Auch andere Gestaltungen, beispielsweise mit einer Quetschflasche, die gleichzeitig Flüssigkeitsspeicher und Fördereinrichtung ist, sind möglich.

Der Flüssigkeitsspender der Fig. 1 und 2 ist ein Tropfenspender und verfügt als solcher die Austragöffnung 108 umgebend über eine Tropfenbildungsgeometrie 110, beispielsweise eine plane oder konkave ringförmige Fläche, an der ein Tropfen in einer Überkopflage des Spenders anhaften kann, bis er sich schwerkraftbedingt von der Tropfenbildungsgeometrie 110 löst.

Der Flüssigkeitsspender 100 verfügt über eine Schutzkappe 10, welche als belüftete Schutzkappe ausgebildet ist. Dies bedeutet, dass ein Kappeninnenraum 14 mit einer umgebenden Atmosphäre über eine Belüftungsdurchbrechung 16 verbunden ist, welche im Falle der Ausgestaltung dieses Ausführungsbeispiels an einer Stirnfläche der Kappenwandung 12 der Schutzkappe 10 vorgesehen ist. Die Belüftungsdurchbrechung 16 ist mit einer Flächenstruktur 18 versehen, wobei es sich um eine flexible Flächenstruktur handelt, die aus mehreren flexiblen Lagen mit unterschiedlichen Eigenschaften zusammengesetzt ist, wie im Weiteren noch erläutert wird. Die in Fig. 2 dargestellte exemplarische Flächenstruktur 18 weist zwei Lagen auf, von denen eine innere Lage 22 als saugfähiges Pad ausgebildet ist und in der Lage ist, nach Aufsetzen der Schutzkappe 10 auf die Haupteinheit 102 einen im Bereich der Tropfenbildungsgeometrie 110 verbliebenen Resttropfen aufzunehmen und gegebenenfalls durch antibakterielle Ausgestaltung darin enthaltene Keime abzutöten. Die der Austragöffnung 108 abgewandte Seite der Flächenstruktur 18 wird durch eine äußere Lage 20 gebildet, die beispielsweise als Sterilfilter ausgebildet sein kann, so dass Keimeintrag in den Kappeninnraum 14 bei aufgesetzter Schutzkappe 10 vermieden wird.

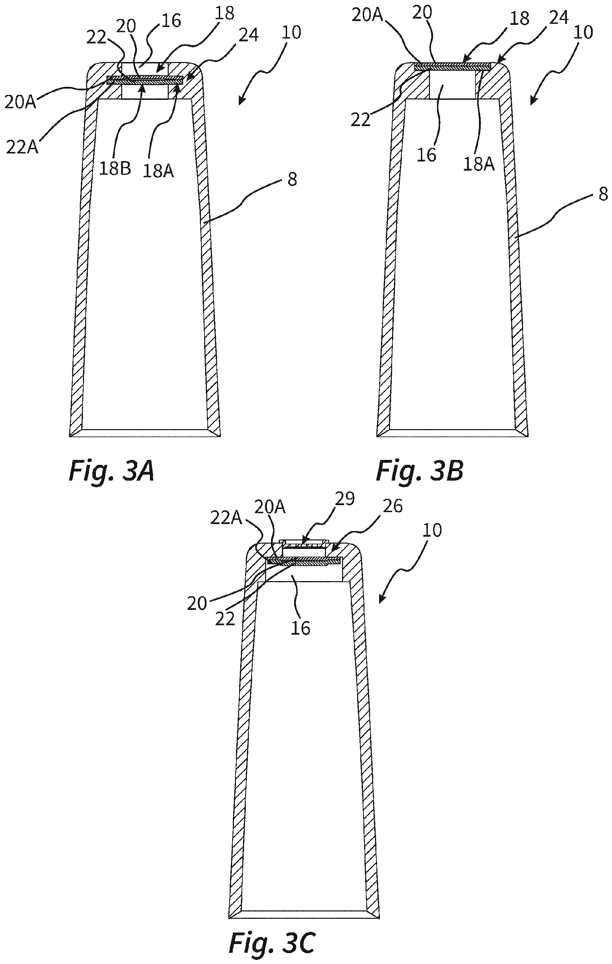

Die Fig. 3A bis 3C zeigen drei Varianten der Schutzkappe 10, von denen die Varianten der Fig. 3A und 3C im Weiteren noch in Hinblick auf ihre jeweilige Herstellung näher erläutert sind.

Bei der Gestaltung gemäß Fig. 3A weist die Schutzkappe 10 einen Grundkörper 8 auf, der sowohl die Mantelflächen der Schutzkappe 10 als auch eine Tragstruktur 24 bildet, die die Belüftungsdurchbrechung 16 umgebend vorgesehen ist, und die sich beidseitig der Flächenstruktur 18 über diese erstreckt und somit die beiden Lagen 20, 22 sichert. Die Lagen 20, 22 sind untereinander nicht fest miteinander verbunden. In Fig. 3A ist zur Verdeutlichung dessen ein leichter Abstand zwischen den Lagen 20, 22 eingezeichnet. In der Praxis werden die Lagen 20, 22 jedoch zumindest in ihren Randbereichen 20A, 22A, insbesondere jedoch vollständig aneinander anliegen. Sie sind jedoch zumindest in einem Zentralbereich nicht unmittelbar miteinander verbunden und werden, wie im Weiteren noch erläutert wird, üblicherweise nicht vor Anbringung am Grundkörper der Schutzkappe miteinander verbunden. Erst im montierten Zustand, wie er beispielsweise in Fig. 3A dargestellt ist, sind die Lagen 20, 22 durch die Tragstruktur 24 aneinander gesichert.

Bei der Variante gemäß Fig. 3B ist die Tragstruktur 24 anders ausgestaltet, da sie sich nur auf einer Seite, vorliegend unterhalb der Flächenstruktur 18, über deren Randbereich 18A erstreckt. Oberhalb der Flächenstruktur 18 überragt die Tragstruktur 24 die Lagen 20, 22 jedoch nicht. Stattdessen sind die Lagen 20, 22, insbesondere jedoch die äußere Lage 20, am Grundkörper 8 der Schutzkappe dadurch befestigt, dass dieser in der im Weiteren noch erläuterten Art und Weise an die zuvor in einer Kavität platzierte Lage 20 angespritzt wird, wobei ein Randbereich 20A der Lage 20 stoffschlüssig mit dem Grundkörper 8 verbunden wird.

Bei der Gestaltung gemäß Fig. 3C ist wiederum keine beidseitig der Flächenstruktur 18 vorgesehene Tragstruktur vorgesehen. Stattdessen ist eine Tragstruktur 26 mit einer Befestigungsfläche 28 lediglich oberhalb der Flächenstruktur 18 vorgesehen. Wie durch die dünneren Randbereiche in Fig. 3C verdeutlicht wird, sind die Lagen 20, 22 der Flächenstruktur 18 hier mittels eines Prägestempels gemeinsam oder nacheinander an die genannte Befestigungsfläche 28 thermisch gebunden worden.

Eine Besonderheit bei der Gestaltung der Fig. 3C besteht in einer als separates Bauteil ausgebildeten Schutzstruktur 29, die an der Außenseite in die Belüftungsdurchbrechung 16 eingeschoben ist und dort kraftschlüssig hält. Diese Schutzstruktur schützt die Flächenstruktur und verhindert insbesondere eine Verletzung einer als Sterilfilter wirkenden Lage. Die Schutzstruktur 29 ist nur exemplarisch bei der Gestaltung der Fig. 3C dargestellt und könnte ebenso auch bei den anderen hier beschriebenen Kappengestaltungen vorgesehen sein.

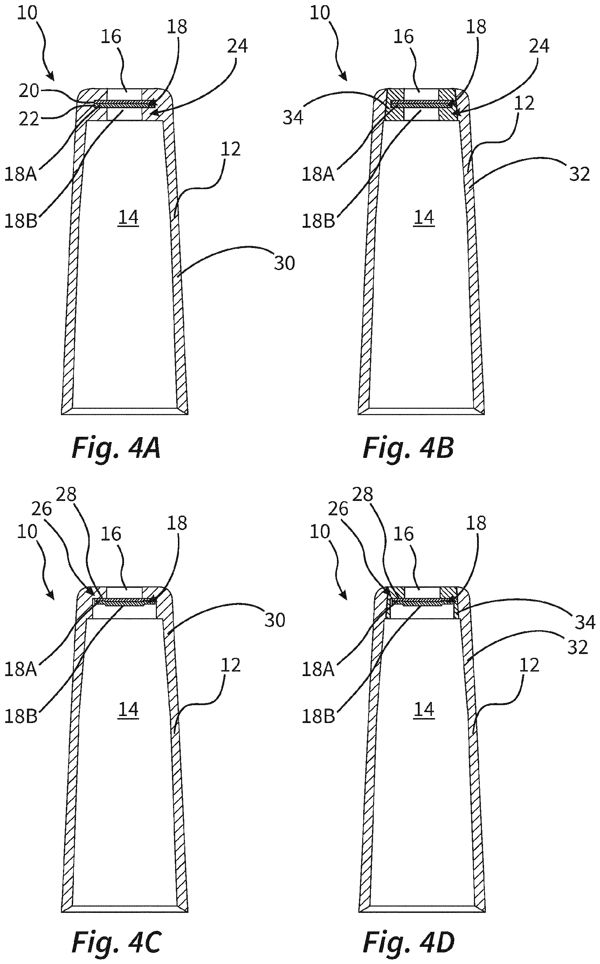

Die Fig. 4A bis 4D zeigen im Hinblick auf die Gestaltungen der Fig. 3A und 3C jeweils zwei unterschiedliche Bauformen. Die Gestaltungen der Fig. 4A und 4C entsprechen dabei denen der Fig. 3A und 3C.

Die Variante der Fig. 4B hingegen zeigt eine Gestaltung, bei der an einem Hauptbauteil 32 der Schutzkappe 10, welches insbesondere auch die Mantelwandungen bildet, ein Belüftungseinsatz 34 stirnseitig angebracht ist, der seinerseits die Belüftungsdurchbrechung 16 und die Flächenstruktur 18 aufweist. Der Belüftungseinsatz 34 ist mittels einer Presspassung im Hauptbauteil 32 der Schutzkappe 10 befestigt. Hier sind auch alternative Befestigungen wie Klebverbindungen, Schweißverbindungen und Gewindeverbindungen möglich.

Entsprechend der Variante der Fig. 4B ist auch bei der Variante der Fig. 4D ein separater Belüftungseinsatz 34 vorgesehen, der die Tragstruktur 26 mit der Befestigungsfläche 28 zur Verfügung stellt und der in gleicher Weise wie der Belüftungseinsatz 34 der Fig. 4B mittels einer Presspassung in das Hauptbauteil 32 der Schutzkappe 10 eingesetzt ist.

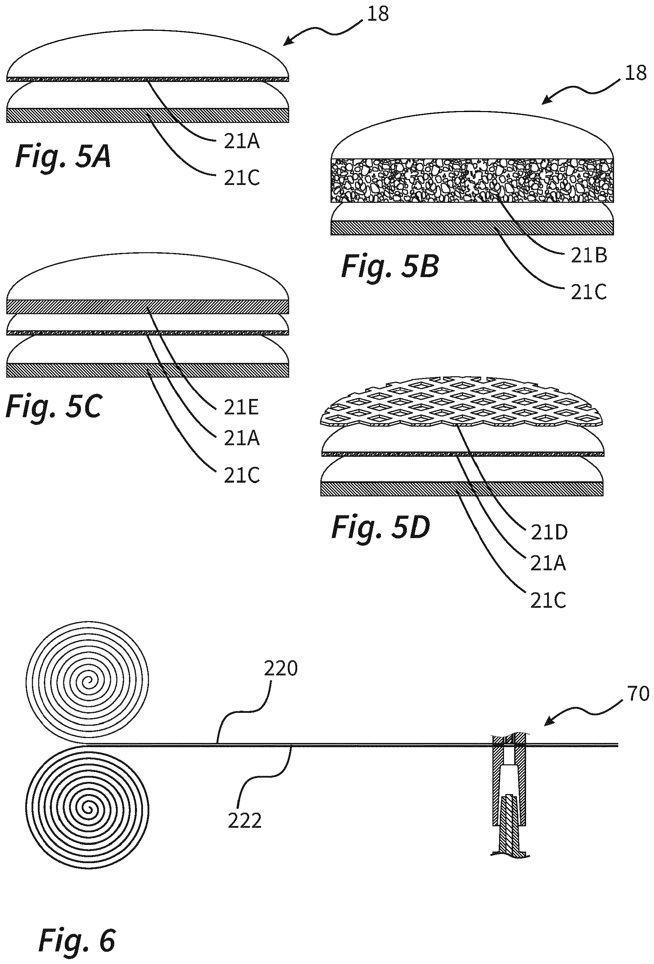

Die Fig. 5A bis 5D verdeutlichen verschiedene Konfigurationen von Einzellagen, die gemeinsam die Flächenstruktur 18 bilden. Da erfindungsgemäß vorgesehen ist, dass die Lagen der Flächenstruktur miteinander nicht oder zumindest nicht in einem Zentralbereich verbunden sind, ist es in vorteilhafter Art und Weise einfach möglich, für besondere Anwendungsfälle besondere Kombinationen von Lagen vorzusehen. Eine technisch aufwändige und nur bei großen Stückzahlen wirtschaftliche Herstellung eines Verbundbandes, aus dem die Flächenstruktur entnommen wird, ist daher nicht erforderlich. Die hier exemplarisch gezeigten Konfigurationen weisen jeweils einen Sterilfilter auf. Die Trenngrenze dieser Sterilfilter beträgt vorzugsweise maximal 1 µ, vorzugsweise maximal 0,5 µm. Auch eine noch feinere Trenngrenze von maximal 0,5 µm kann vorteilhaft sein.

Die Fig. 5A zeigt eine Gestaltung, bei der ein Sterilfilter 21A und eine saugfähige Lage 21C gemeinsam die Flächenstruktur 18 bilden. Dabei ist der Sterilfilter 21A recht dünn dargestellt, um zu verdeutlichen, dass es sich in diesem Fall um einen Membranfilter handelt, also einen Filter, der eine Vielzahl von Poren umfasst, die jeweils kleiner als eine vorgegebene Trenngrenze sind, um bestimmte Bestandteile wie Bakterien nicht hindurchzulassen.

Bei der Variante gemäß Fig. 5B ist ebenfalls ein Sterilfilter 21B vorgesehen. Dieser ist jedoch als Tiefenfilter ausgestaltet. Dies bedeutet, dass zwar nicht jede einzelne Pore des Filters kleiner als die beabsichtigte Trenngrenze ist, der Filter aufgrund seiner Dicke jedoch dennoch in der Lage ist, Bestandteile, die größer als die Trenngrenze sind, zuverlässig herauszufiltern.

Während beim Membranfilter 21A der Fig. 5A die durch den Filter abgeschiedenen Bestandteile an der Oberseite des Filters verbleiben, gelangen sie beim Tiefenfilter 21B der Fig. 5B in den Filter selbst hinein und werden dort von der hindurchtretenden Luft abgeschieden.

Die Gestaltung der Fig. 5C sieht vor, dass zusätzlich zu einem Sterilfilter 21A und einem saugfähigen Pad 21C eine Sichtschutzlage 21E vorgesehen ist. Diese erfüllt primär die Funktion, den Sterilfilter 21A zu kaschieren. Da der Sterilfilter 21A üblicherweise eine andere Farbgebung als die Schutzkappe 10 im Übrigen hat, hat sich herausgestellt, dass Benutzer fälschlicherweise davon ausgehen, der Sterilfilter 21A sei bei Inbetriebnahme des Spenders zu beseitigen. Durch die genannte Sichtschutzlage 21E, die in gleicher Farbgebung wie die Schutzkappe 10 im Übrigen ausgebildet ist, wird das Vorhandensein eines deutlich anders gefärbten Sterilfilters 21A kaschiert, so dass der Benutzer nicht auf die Idee kommt, die Flächenstruktur 18 zu verletzen.

Bei der Gestaltung gemäß Fig. 5D sind wiederum eine Sterilfilterlage 21A und eine saugfähige Lage 21C zur Aufnahme eines Resttropfens vorgesehen. Hier ist jedoch weiterhin eine Stützlage 21D vorgesehen, die vergleichsweise fest ist und somit den Sterilfilter 21A mechanisch gegen versehentliche oder mutwillige Verletzungen schützt.

Die Fig. 6 bis 7H zeigen ein erstes Verfahren zur Herstellung einer Schutzkappe der beschriebenen Art, vorliegend jener Schutzkappe, die auch in den Fig. 3A und 4A dargestellt ist.

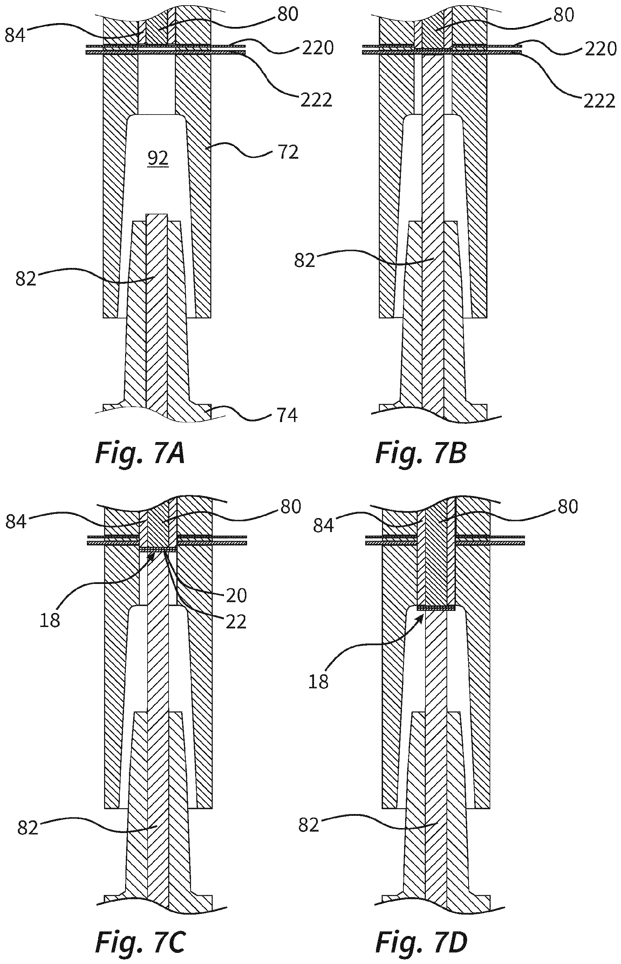

In Fig. 6 ist schematisch der grundsätzliche Aufbau gezeigt. Zwei Bänder 220, 222 werden einem kombinierten Stanz- und Spritzgusswerkzeug 70 zugeführt. Die Fig. 7A bis 7H zeigen die Verarbeitung dieser Bänder 220, 222 und die Herstellung der Schutzkappe 10 im Bereich dieses Stanz- und Spritzgusswerkzeugs 70.

Fig. 7A zeigt einen Ausgangszustand. In diesem ist eine Kavität 90, die primär durch eine Oberschale 72 und eine Unterschale 74 begrenzt wird, noch geöffnet. Die zuvor beschriebenen Bänder 220, 222 sind in zwei Schlitze der Oberschale 72 eingefahren. Die Unterschale 74 ist zentrisch mit einem vertikal verfahrbaren Stempel 82 in einer Durchbrechung der Unterschale 74 vorgesehen. Entsprechend ist die Oberschale 72 mit einem ebenfalls vertikal verfahrbaren Stempel 80 sowie einer unabhängig hiervon beweglichen und den Stempel 80 umgebenden Schneidkontur 84 versehen. Der Stempel 80 und die Schneidkontur 84 sind in einer Durchbrechung der Oberschale 72 angeordnet.

Im Ausgangszustand der Fig. 7A befinden sich der Stempel 80 und die Schneidkontur 84 oberhalb der Schlitze, durch die die Bänder 220, 222 zugeführt sind.

Hiervon ausgehend wird zunächst der Stempel 82 vertikal von unten nach oben verfahren, bis er an der Unterseite der unteren Bahn 222 anliegt. Der obere Stempel 80 und die Schneidkontur 84 drücken gleichzeitig von oben auf das Band 220, so dass die Bänder 220, 222 aneinandergedrückt werden. Es stellt sich der Zustand der Fig. 7B ein.

In der durch Fig. 7C verdeutlichten Weise fahren dann die Stempel 80, 82 sowie die Schneidkontur 84 gemeinsam vertikal nach unten, wodurch die Flächenstruktur 18 bestehend aus zwei Lagen 20, 22 von den umgebenden Bändern 220, 222 getrennt werden, indem die Schneidkontur 84 sie am Rand der Durchbrechung in der Oberschale 72 abschert. Gemeinsam fahren die Stempel 80, 82 und die Schneidkontur 84 bis in die Lage hinab, die in Fig. 7D dargestellt ist.

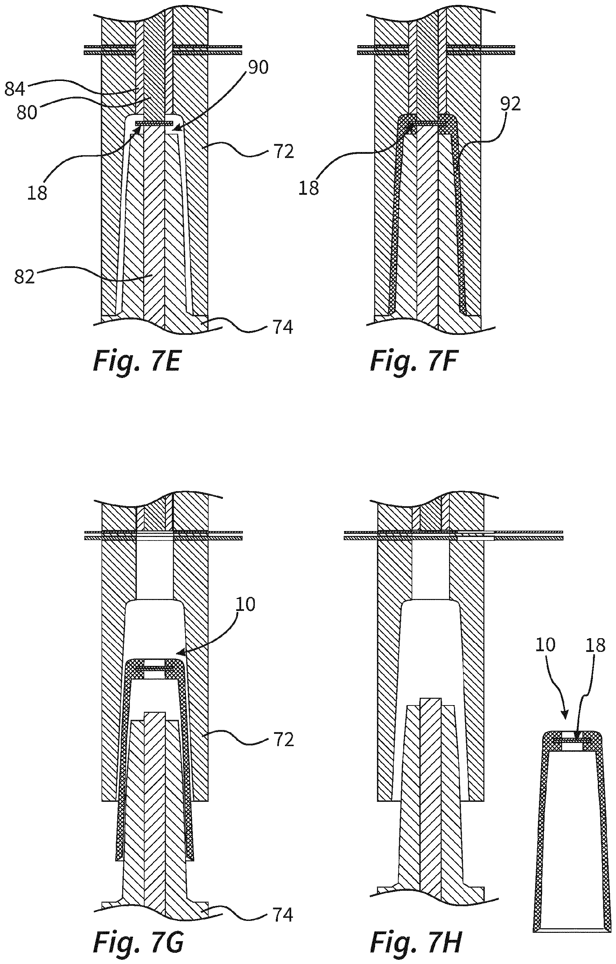

Ausgehend hiervon fahren die Stempel 80, 82 noch etwas weiter, während gleichzeitig oder getrennt hiervon die Unterschale 74 und die Oberschale 72 aufeinander zu verfahren werden, so dass die Kavität 90 geschlossen wird. Beim Absenken der Stempel 80, 82 in die Position der Fig. 7E wird die Schneidkontur 84 nicht mehr mitbewegt, so dass es erstmals zu einer Relativverlagerung zwischen der Schneidkontur 84 und dem Stempel 80 kommt. Die Schneidkontur 84 bleibt weiter oben und bildet mit ihrer nach unten weisenden Stirnseite damit einen Teil der begrenzenden Wandung der Kavität 90.

Obwohl der genannte Schritt, in dem die Stempel 80, 82 gegenüber der Schneidkontur relativverlagert werden, als vorteilhaft angesehen wird, ist er nicht zwingend. Die zuvor beschriebene Gestaltung der Fig. 3B kann mittels eines Verfahrens erzeugt werden, was dem hier beschriebenen Verfahren weitgehen gleicht, jedoch die genannte Relativverlagerung nicht vorsieht. Für ein solches Verfahren kann ein Werkzeug vorgesehen sein, bei dem die Schneidkontur 84 und der Stempel 80 als gemeinsames Bauteil ausgebildet sind.

Ausgehend vom Zustand der Fig. 7E wird dann flüssiger Kunststoff, beispielsweise PP oder HDPE, durch eine nicht dargestellte Zuführöffnung in die Kavität 90 geleitet, wie in Fig. 7F ersichtlich ist. In der Kavität 90 härtet der Kunststoff 92 aus, so dass anschließend in der durch die Fig. 7G und 7H verdeutlichten Weise nach Beabstandung der Unterschale 74 und der Oberschale 72 die fertiggestellte Schutzkappe 10 entnommen werden kann.

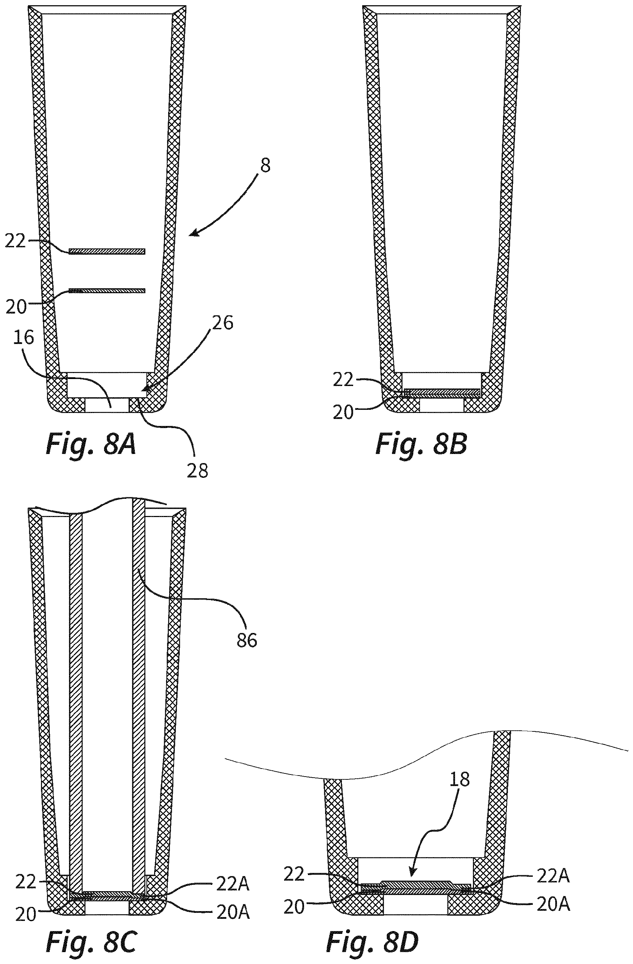

Ein alternatives Verfahren ist in den Fig. 8A bis 8D dargestellt. Dieses Verfahren geht von einem bereits durch Spritzguss fertig hergestellten Grundkörper 8 der Schutzkappe 10 aus, in dem stirnseitig eine Belüftungsdurchbrechung 16 vorgesehen ist. Diese durchbricht eine Tragstruktur 26, welche primär durch eine Befestigungsfläche 28 gebildet wird.

In diesen Grundkörper 8 werden die beiden Lagen 20, 22 der Flächenstruktur 18 einzeln eingefügt. Diese sind zu diesem Zeitpunkt nicht fest miteinander verbunden, können jedoch natürlich abweichend von Fig. 8A in bereits aufeinanderliegender Form eingebracht werden. Die beiden Lagen 20, 22 weisen eine Fläche auf, die größer als die Querschnittsfläche der Belüftungsdurchbrechung 16 ist, so dass sie auf der Befestigungsfläche 28 zum Aufliegen kommen, wie Fig. 8B zeigt.

In der durch Fig. 8C verdeutlichten Weise wird anschließend ein erhitzter Prägestempel 86 von oben in den Grundkörper 8 eingeführt. Die ringförmige Stirnfläche des Prägestempels wird dafür verwendet, die Randbereiche 20A, 22A der Lagen 20, 22 gegen die Befestigungsfläche 28 zu pressen und sie dabei thermisch miteinander und mit der Befestigungsfläche 28 zu verbinden. Ergebnis ist die zweilagige Struktur, die in Fig. 8D ersichtlich ist.

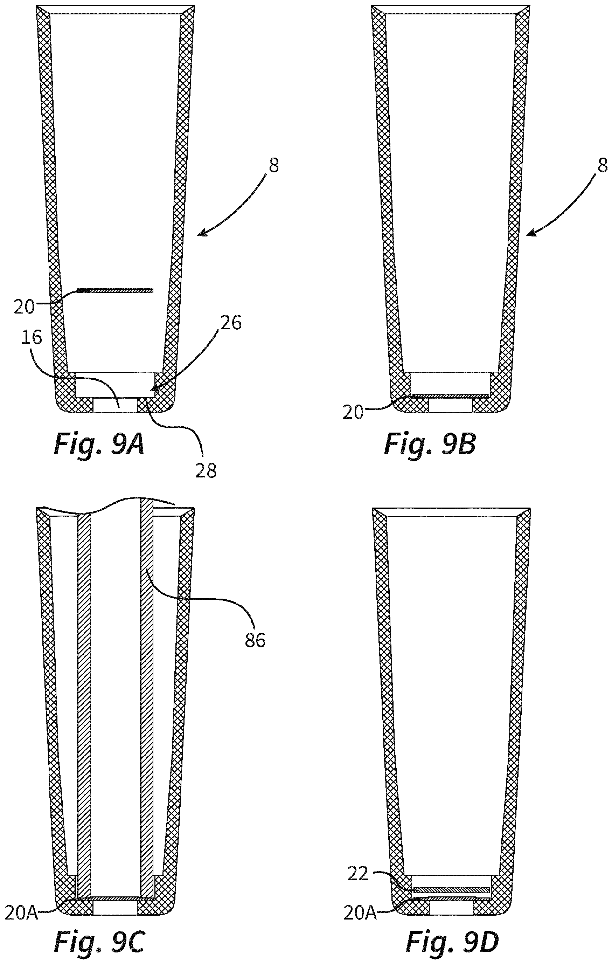

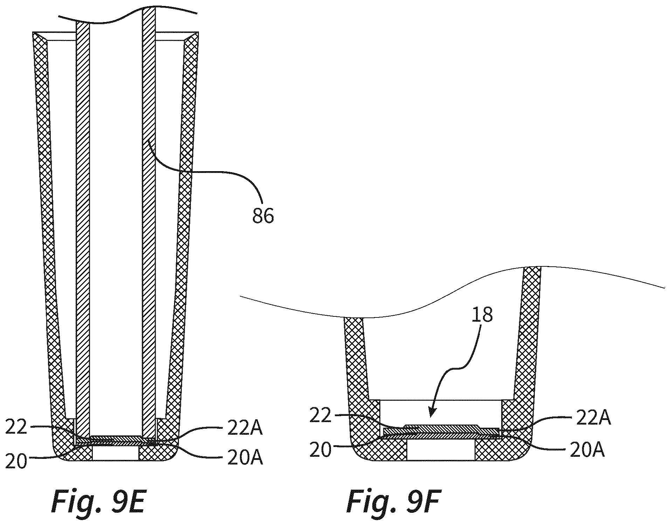

Ein hierzu alternatives Verfahren ergibt sich aus den Fig. 9A bis 9F. Hier werden die beiden Lagen 20, 22 nacheinander eingefügt. Wie sich aus den Fig. 9A bis 9C ergibt, wird zunächst eine erste Lage 20 auf der Befestigungsfläche 28 platziert und mittels des Prägestempels 86 thermisch dort befestigt. Erst anschließend erfolgt die Einbringung der zweiten Lage 22, die anschließend in der durch Fig. 9E verdeutlichten Weise ebenfalls mittels des erhitzten Prägestempels 86 befestigt wird, nämlich auf der zuvor bereits befestigten Lage 20.

Wie Fig. 9F zeigt, ergibt sich hieraus ein ähnlicher Aufbau wie beim Verfahren der Fig. 8A bis 8D. Wenngleich das Verfahren der Fig. 9A bis 9F mehr Fertigungsschritte umfasst, lässt sich hiermit je nach Material der Lagen 20, 22 eine bessere Dichtheit im Randbereich erzielen, so dass auch dieses komplexere Verfahren seine Daseinsberechtigung hat.

## Patentansprüche

1. Belüftete Schutzkappe (10) für einen Flüssigkeitsspender (100) zum Austrag einer pharmazeutischen Flüssigkeit mit den folgenden Merkmalen:
a. die Schutzkappe (10) weist eine Kappenwandung (12) auf, die einen Kappeninnenraum (14) umgibt, und
b. die Kappenwandung (12) weist eine Belüftungsdurchbrechung (16) auf, die von einer flexiblen Flächenstruktur (18) überspannt wird, und
c. ein Randbereich (18A) der Flächenstruktur (18) ist umlaufend an einem Rand der Belüftungsdurchbrechung (16) fixiert, und
d. die Schutzkappe (10) ist auf eine Haupteinheit (102) des Flüssigkeitsspenders (100) aufsetzbar und schützt eine Austragöffnung (108) der Haupteinheit (102) im aufgesetzten Zustand, **gekennzeichnet durch** das folgende Merkmal:
e. die Flächenstruktur (18) besteht aus mindestens zwei luftdurchlässigen Lagen (20, 22), die unmittelbar aneinander anliegen, jedoch zumindest in einem Zentralbereich (18B) der Flächenstruktur (18) nicht fest miteinander verbunden sind, und
f. die Flächenstruktur (18) überspannt die Belüftungsdurchbrechung (16) und gewährleistet so, dass ein Luftaustausch zwischen einem Kappeninnenraum (14) und einer Umgebung bei aufgesetzter Schutzkappe (10) nur durch die Flächenstruktur (18) hindurch möglich ist.

2. Belüftete Schutzkappe (10) nach Anspruch 1 mit dem folgenden weiteren Merkmal:
a. die zwei Lagen (20, 22) der Flächenstruktur (18) sind durch eine gemeinsame angespritzte Tragstruktur (24) am Rand der Belüftungsdurchbrechung (16) gehalten, die einseitig oder einstückig beidseitig der Flächenstruktur über den Randbereich (18A) der Flächenstruktur (18) hinüberragt.

3. Belüftete Schutzkappe (10) nach Anspruch 1 mit den folgenden weiteren Merkmalen:
a. am Rand der Belüftungsdurchbrechung (16) ist eine Tragstruktur (26) mit einer ringförmigen Befestigungsfläche (28) vorgesehen, und
b. eine erste Lage (20) der Flächenstruktur ist in einem Randbereich (20A) thermisch an der Befestigungsfläche (28) befestigt, und
c. eine zweite Lage (22) der Flächenstruktur (18) ist in einem Randbereich (22A) thermisch am Randbereich (20A) der ersten Lage (20) befestigt.

4. Belüftete Schutzkappe (10) nach einem der vorstehenden Ansprüche mit dem folgenden weiteren Merkmal:
a. die Schutzkappe (10) weist ein Hauptbauteil (30) auf, welches mindestens zum überwiegenden Teil eine Mantelwandung der Kappenwandung (12) bildet und an dem die Tragstruktur (24; 26) einstückig vorgesehen ist.

5. Belüftete Schutzkappe nach einem der Ansprüche 1 bis 3 mit den folgenden weiteren Merkmalen:
a. die Schutzkappe (10) weist ein Hauptbauteil (32) auf, welches mindestens zum überwiegenden Teil eine Mantelwandung der Kappenwandung (12) bildet und eine Öffnung zur Aufnahme eines Belüftungseinsatzes (34) aufweist, und
b. die Schutzkappe (10) weist einen in die Öffnung dichtend eingesetzten Belüftungseinsatz (34) auf, der die umgebende Tragstruktur (24; 26) und die hiervon gehaltene und umgebene Flächenstruktur (18) umfasst.

6. Belüftete Schutzkappe (10) nach einem der vorstehenden Ansprüchen mit mindestens einem der folgenden weiteren Merkmale:
a. mindestens eine Lage der Flächenstruktur (18) wird durch einen Sterilfilter (21A, 21B) gebildet, der vorzugsweise eine Trenngrenze von maximal 1 µm aufweist, insbesondere vorzugsweise eine Trenngrenze von maximal 0,5 µm oder von maximal 0,2 µm, und/oder
b. mindestens eine Lage der Flächenstruktur (18) ist eine saugfähige Lage (21C), die zur Aufnahme eines Resttropfens von einer Austragöffnung des Spenders vorgesehen ist, wobei diese ausgehend vom Kappeninnenraum (14) vorzugsweise die erste Lage der Flächenstruktur (18) darstellt, und/oder
c. mindestens eine Lage der Flächenstruktur (18) ist als Tiefenfilter (21B) ausgebildet, und/oder
d. mindestens eine Lage der Flächenstruktur (18) ist als Membranfilter (21A) ausgebildet, und/oder
e. mindestens eine Lage der Flächenstruktur (18) weist eine hydrophile Oberfläche auf, und/oder
f. mindestens eine Lage der Flächenstruktur (18) weist eine hydrophobe Oberfläche auf, und/oder
g. mindestens eine Lage der Flächenstruktur (18) ist als antibakterielle Lage (21C) ausgebildet und weist einen bakterientötenden Inhaltsstoff auf, und/oder
h. mindestens eine Lage der Flächenstruktur (18) ist als Stützlage (21D) ausgebildet, insbesondere eine äußerste Lage, und/oder
i. mindestens eine äußerste Lage ist als Sichtschutzlage (21E) ausgebildet und weist eine mit der umgebenden Tragstruktur einheitliche Farbe auf, und/oder
j. die Schutzkappe (10) weist eine Schutzstruktur zum Schutz der Flächenstruktur auf, wobei die Schutzstruktur (29) als von der Tragstruktur (24) getrenntes Bauteil ausgebildet ist und mit der Tragstruktur (24) kraftschlüssig oder formschlüssig verbunden ist.

7. Belüftungseinsatz (34) für einen Belüftungskanal zur Verwendung in einer Schutzkappe (10) nach nach Anspruch 5 und zum Schutz einer Austragöffnung (108) einer Haupteinheit (102) eines Flüssigkeitsspenders (100) im aufgesetzten Zustand der Schutzkappe (108), mit den folgenden Merkmalen:
a. der Belüftungseinsatz (34) weist eine Belüftungsdurchbrechung (16) auf, die von einer Tragstruktur (24; 26) umgeben ist, und
b. die Belüftungsdurchbrechung (16) ist von einer flexiblen Flächenstruktur (18) überspannt, und
c. ein Randbereich (18A) der Flächenstruktur (18) ist umlaufend an der Tragstruktur (24; 26) fixiert,
**gekennzeichnet durch** das folgende weitere Merkmal:
d. die Flächenstruktur (18) besteht aus mindestens zwei luftdurchlässigen Lagen (20, 22), die unmittelbar aneinander anliegen, jedoch zumindest in einem Zentralbereich (18B) der Flächenstruktur (18) nicht fest miteinander verbunden sind, und
e. die Flächenstruktur (18) überspannt die Belüftungsdurchbrechung (16) und gewährleistet so, dass ein Luftaustausch zwischen einem Kappeninnenraum (14) und einer Umgebung bei aufgesetzter Schutzkappe (10) nur durch die Flächenstruktur (18) hindurch möglich ist.

8. Belüftungseinsatz nach Anspruch 7 mit dem folgenden weiteren Merkmal:
a. die zwei Lagen (20, 22) der Flächenstruktur (18) sind durch eine gemeinsame angespritzte Tragstruktur (24) am Rand der Belüftungsdurchbrechung (16) gehalten, die einseitig oder einstückig beidseitig der Flächenstruktur über den Randbereich (18A) der Flächenstruktur (18) hinüberragt.

9. Belüftungseinsatz nach Anspruch 7 mit den folgenden weiteren Merkmalen:
a. am Rand der Belüftungsdurchbrechung (16) ist eine Tragstruktur (26) mit einer ringförmigen Befestigungsfläche (28) vorgesehen, und
b. eine erste Lage (20) der Flächenstruktur (18) ist in einem Randbereich (20A) thermisch mit der Befestigungsfläche (28) verbunden, und
c. eine zweite Lage (22) der Flächenstruktur (18) ist in einem Randbereich (22A) thermisch mit dem Randbereich (20A) der ersten Lage (20) verbunden.

10. Belüftungseinsatz nach einem der Ansprüche 7 bis 9 mit einem der folgenden Merkmale:
a. der Belüftungseinsatz (34) weist eine sich verjüngende Mantelfläche zur vereinfachten Montage in den Belüftungskanal auf, oder
b. der Belüftungseinsatz (34) ist mittels einer Schnappverbindung im Belüftungskanal befestigt, oder
c. der Belüftungseinsatz (34) ist mittels einer Gewindeverbindung im Belüftungskanal befestigt, oder
d. der Belüftungseinsatz (34) ist mittels einer Kleb- oder Schweißverbindung im Belüftungskanal befestigt.

11. Flüssigkeitsspender (100) für pharmazeutische Flüssigkeiten, mit den folgenden Merkmalen:
a. der Flüssigkeitsspender (100) verfügt über eine Haupteinheit (102) mit einem Flüssigkeitsspeicher (104), einer Fördereinrichtung (106) und einer Austragöffnung (108) zur Abgabe der Flüssigkeit, und
b. der Flüssigkeitsspender (100) verfügt über eine Schutzkappe (10), die auf die Haupteinheit (102) aufsetzbar ist und die Austragöffnung (108) im aufgesetzten Zustand schützt,
**gekennzeichnet durch** eines der Merkmale:
c. die Schutzkappe (10) ist nach einem der Ansprüche 1 bis 6 ausgebildet und/oder
d. die Schutzkappe (10) umfasst einen Belüftungseinsatz (34) nach Anspruch 7 bis 10.

12. Flüssigkeitsspender (100) nach Anspruch 11 mit den folgenden weiteren Merkmalen:
a. mindestens eine Lage (22) der Flächenstruktur (18) ist eine saugfähige oder antibakterielle ausgestaltete Lage (22), wobei diese Lage bei aufgesetzter Schutzkappe (10) unmittelbar oberhalb der Austragöffnung (108) angeordnet ist, so dass ein an der Austragöffnung verbliebener Resttropfen hiervon aufgenommen und/oder dekontaminiert werden kann.

13. Flüssigkeitsspender (100) nach Anspruch 11 oder 12 mit mindestens einem der folgenden weiteren Merkmale:
a. der Flüssigkeitsspender (100) ist als Tropfenspender ausgebildet und weist die Austragöffnung (108) umgebend eine Tropfenbildungsgeometrie (110) auf, und/oder
b. der Flüssigkeitsspender (100) ist mit einer pharmazeutischen Flüssigkeit befüllt, und/oder
c. der Flüssigkeitsspender (100) weist eine Pumpeinrichtung (106) zur Förderung der Flüssigkeit aus dem Flüssigkeitsspeicher (104) zur Austragöffnung (108) auf, und/oder
d. der Flüssigkeitsspender (100) weist eine Quetschflasche (112) auf, die zum Zwecke der zur Förderung der Flüssigkeit aus dem Flüssigkeitsspeicher (104) zur Austragöffnung (108) zusammendrückbar ist, und/oder
e. der Flüssigkeitsspender (100) weist einen Druckspeicher auf, und/oder
f. der Flüssigkeitsspender (100) ist als Sprühspender ausgebildet, mittels dessen die Flüssigkeit in zerstäubter Form abgegeben wird.

14. Verfahren zur Herstellung einer Schutzkappe (10) nach einem der Ansprüche 2 und 4 bis 7 oder eines Belüftungseinsatzes nach einem der Ansprüche 8 oder 10 mit den folgenden Schritten:
a. mindestens eine erste Lage eines ersten flächigen Materials und eine zweite Lage eines zweiten flächigen Materials werden kontinuierlich in Form flächiger Bänder (220, 222) zugeführt, und
b. in einem überlappenden Bereich der Bänder werden die mindestens zwei Lagen durch einen oberseitigen Stempel (80) und einen unterseitigen Stempel (82) zusammengedrückt und es wird die Stempel (80, 82) umgebend mit einer Schneidkontur (84) ein Schnitt vorgenommen, mit dem die zwei Lagen (20, 22) einer für die Schutzkappe (10) oder den Belüftungseinsatz (34) vorgesehenen Flächenstruktur (18) von den flächigen Bändern (220, 222) getrennt wird, und
c. die mehrlagige Flächenstruktur (18) wird in durch die Stempel (80, 82) fixiertem Zustand in eine Spritzgusskavität (90) eingefügt, und
d. es wird ein Kunststoffmaterial (92) in die Spritzgusskavität (90) eingespritzt, welches eine gemeinsame Tragstruktur (24) bildet, die einseitig oder einstückig beidseitig der Flächenstruktur (18) über den Randbereich (18A) der Flächenstruktur (18) hinüberragt und die Lagen (20, 22) der Flächenstruktur fixiert, und
e. die beidseitigen Stempel (80, 82) werden nach Aushärtung der Tragstruktur (24) von der Flächenstruktur (18) beabstandet.

15. Verfahren zur Herstellung einer Schutzkappe (10) nach einem der Ansprüche 3 bis 7 oder eines Belüftungseinsatzes nach einem der Ansprüche 9 oder 10 mit den folgenden Schritten:
a. es wird ein Grundkörper (8) einer belüfteten Schutzkappe oder eines Belüftungseinsatz zur Verfügung gestellt, der über eine Belüftungsdurchbrechung verfügt, die von einer Tragstruktur (26) mit einer ringförmigen Befestigungsfläche (28) umgeben ist, und
b. eine erste Lage (20) der Flächenstruktur und eine zweite Lage (22) der Flächenstruktur werden derart platziert, dass der Randbereich (20A) der ersten Lage (20) in Kontakt mit der ringförmigen Befestigungsfläche (28) gelangt und dass der Randbereich (22A) der zweiten Lage (22) in Kontakt mit dem Randbereich (20A) der ersten Lage (20) gelangt, und
c. es wird mittels eines erhitzten Stempels (86) nach Einfügung der zweiten Lage (22) eine thermische Fügung der Randbereiche (20A, 22A) der ersten Lage (20) und zweiten Lage (22) untereinander bewirkt.

16. Verfahren nach Anspruch 15 mit dem folgenden Merkmal:
a. bereits nach der Platzierung der ersten Lage (20) und vor Platzierung der zweiten Lage (22) wird mittels eines erhitzten Stempels (86) der Randbereich (20A) der ersten Lage thermisch mit der ringförmigen Befestigungsfläche (28) verbunden.

## Claims

1. Vented protective cap (10) for a liquid dispenser (100) for discharging a pharmaceutical liquid, having the following features:
a. the protective cap (10) has a cap wall (12) which surrounds a cap interior (14), and
b. the cap wall (12) has a venting aperture (16) which is spanned by a flexible sheet-like structure (18), and
c. a boundary region (18A) of the sheet-like structure (18) is fixed in an encircling manner to a boundary of the venting aperture (16), and
d. the protective cap (10) is able to be mounted onto a main unit (102) of the liquid dispenser (100) and protects a discharge opening (108) of the main unit (102) in the mounted state, **characterized by** the following feature:
e. the sheet-like structure (18) consists of at least two air-permeable layers (20, 22) which bear directly on each other but, at least in a central region (18B) of the sheet-like structure (18), are not connected fixedly to each other, and
f. the sheet-like structure (18) spans the venting aperture (16) and thus ensures that, with the protective cap (10) mounted, an exchange of air between a cap interior (14) and surroundings is possible only through the sheet-like structure (18).

2. Vented protective cap (10) according to Claim 1, having the following further feature:
a. the two layers (20, 22) of the sheet-like structure (18) are held at the boundary of the venting aperture (16) by a common injection-moulded bearing structure (24) which, on one side or integrally on both sides of the sheet-like structure, projects over the boundary region (18A) of the sheet-like structure (18).

3. Vented protective cap (10) according to Claim 1, having the following further features:
a. a bearing structure (26) having a ring-shaped fastening surface (28) is provided at the boundary of the venting aperture (16), and
b. a first layer (20) of the sheet-like structure is, in a boundary region (20A), thermally bonded to the fastening surface (28), and
c. a second layer (22) of the sheet-like structure (18) is, in a boundary region (22A), thermally bonded to the boundary region (20A) of the first layer (20).

4. Vented protective cap (10) according to one of the preceding claims, having the following further feature:
a. the protective cap (10) has a main component (30) which at least predominantly forms a lateral wall of the cap wall (12) and on which the bearing structure (24; 26) is provided integrally.

5. Vented protective cap according to one of Claims 1 to 3, having the following further features:
a. the protective cap (10) has a main component (32) which at least predominantly forms a lateral wall of the cap wall (12) and which has an opening for receiving a venting insert (34), and
b. the protective cap (10) has a venting insert (34) which is inserted sealingly into the opening and which comprises the surrounding bearing structure (24; 26) and the sheet-like structure (18) held and surrounded by the latter.

6. Vented protective cap (10) according to one of the preceding claims, having at least one of the following further features:
a. at least one layer of the sheet-like structure (18) is formed by a sterile filter (21A, 21B), which preferably has a separation limit of at most 1 µm in size, in particular preferably a separation limit of at most 0.5 µm or of at most 0.2 µm in size, and/or
b. at least one layer of the sheet-like structure (18) is an absorbent layer (21C), which is provided for absorbing a residual drop from a discharge opening of the dispenser, wherein, proceeding from the cap interior (14), said at least one layer preferably constitutes the first layer of the sheet-like structure (18), and/or
c. at least one layer of the sheet-like structure (18) is formed as a deep-bed filter (21B), and/or
d. at least one layer of the sheet-like structure (18) is formed as a membrane filter (21A), and/or
e. at least one layer of the sheet-like structure (18) has a hydrophilic surface, and/or
f. at least one layer of the sheet-like structure (18) has a hydrophobic surface, and/or
g. at least one layer of the sheet-like structure (18) is formed as an antibacterial layer (21C) and comprises a bactericidal constituent, and/or
h. at least one layer of the sheet-like structure (18) is formed as a support layer (21D), in particular an outermost layer, and/or
i. at least one outermost layer is formed as a screening layer (21E) and has a colour which is uniform with respect to the surrounding bearing structure, and/or
j. the protective cap (10) has a protective structure for protecting the sheet-like structure, wherein the protective structure (29) is formed as a component which is separate from the bearing structure (24) and is connected in a force-fitting manner or form-fitting manner to the bearing structure (24).

7. Venting insert (34) for a venting channel, for use in a protective cap (10) according to Claim 5, and for protecting a discharge opening (108) of a main unit (102) of a liquid dispenser (100) in the mounted state of the protective cap (108), having the following features:
a. the venting insert (34) has a venting aperture (16) which is surrounded by a bearing structure (24; 26), and
b. the venting aperture (16) is spanned by a flexible sheet-like structure (18), and
c. a boundary region (18A) of the sheet-like structure (18) is fixed in an encircling manner to the bearing structure (24; 26),
**characterized by** the following further feature:
d. the sheet-like structure (18) consists of at least two air-permeable layers (20, 22) which bear directly on each other but, at least in a central region (18B) of the sheet-like structure (18), are not connected fixedly to each other, and
e. the sheet-like structure (18) spans the venting aperture (16) and thus ensures that, with the protective cap (10) mounted, an exchange of air between a cap interior (14) and surroundings is possible only through the sheet-like structure (18).

8. Venting insert according to Claim 7, having the following further feature:
a. the two layers (20, 22) of the sheet-like structure (18) are held at the boundary of the venting aperture (16) by a common injection-moulded bearing structure (24) which, on one side or integrally on both sides of the sheet-like structure, projects over the boundary region (18A) of the sheet-like structure (18).

9. Venting insert according to Claim 7, having the following further features:
a. a bearing structure (26) having a ring-shaped fastening surface (28) is provided at the boundary of the venting aperture (16), and
b. a first layer (20) of the sheet-like structure (18) is, in a boundary region (20A), thermally bonded to the fastening surface (28), and
c. a second layer (22) of the sheet-like structure (18) is, in a boundary region (22A), thermally bonded to the boundary region (20A) of the first layer (20).

10. Venting insert according to one of Claims 7 to 9, having one of the following features:
a. the venting insert (34) has a tapering lateral surface for simplified fitting into the venting channel, or
b. the venting insert (34) is fastened in the venting channel by means of a snap-action connection, or
c. the venting insert (34) is fastened in the venting channel by means of a threaded connection, or
d. the venting insert (34) is fastened in the venting channel by means of an adhesive or welded connection.

11. Liquid dispenser (100) for pharmaceutical liquids, having the following features:
a. the liquid dispenser (100) has a main unit (102), which has a liquid store (104) and a conveying device (106) and a discharge opening (108) for releasing the liquid, and
b. the liquid dispenser (100) has a protective cap (10) which is able to be mounted onto the main unit (102) and which protects the discharge opening (108) in the mounted state,
**characterized by** one of the features:
c. the protective cap (10) is designed according to one of Claims 1 to 6, and/or
d. the protective cap (10) comprises a venting insert (34) according to Claims 7 to 10.

12. Liquid dispenser (100) according to Claim 11, having the following further features:
a. at least one layer (22) of the sheet-like structure (18) is an absorbent or antibacterial layer (22), wherein said layer, with the protective cap (10) mounted, is arranged directly above the discharge opening (108), with the result that a residual drop remaining at the discharge opening can be absorbed and/or decontaminated by said layer.

13. Liquid dispenser (100) according to Claim 11 or 12, having at least one of the following further features:
a. the liquid dispenser (100) is designed as a drop dispenser and has, in a manner surrounding the discharge opening (108), a drop formation geometry (110), and/or
b. the liquid dispenser (100) is filled with a pharmaceutical liquid, and/or
c. the liquid dispenser (100) has a pump device (106) for conveying the liquid from the liquid store (104) to the discharge opening (108), and/or
d. the liquid dispenser (100) has a squeeze bottle (112), which is able to be compressed for the purpose of conveying the liquid from the liquid store (104) to the discharge opening (108), and/or
e. the liquid dispenser (100) has a pressure store, and/or
f. the liquid dispenser (100) is designed as a spray dispenser, by means of which the liquid is released in atomized form.

14. Method for producing a protective cap (10) according to one of Claims 2 and 4 to 7 or a venting insert according to either of Claims 8 and 10, comprising the following steps:
a. continuously feeding in the form of sheet-like bands (220, 222) at least one first layer of a first sheet-like material and one second layer of a second sheet-like material, and
b. in an overlapping region of the bands, pressing together the at least two layers by way of a top-side stamp (80) and a bottom-side stamp (82) and making a cut using a cutting contour (84) in a manner surrounding the stamps (80, 82), by way of which cut the two layers (20, 22) of a sheet-like structure (18) provided for the protective cap (10) or for the venting insert (34) are separated from the sheet-like bands (220, 222), and
c. inserting the multi-layer sheet-like structure (18) into an injection moulding cavity (90) in a state fixed by the stamps (80, 82), and
d. injecting a plastic material (92) into the injection moulding cavity (90), which plastic material forms a common bearing structure (24) which, on one side or integrally on both sides of the sheet-like structure (18), projects over the boundary region (18A) of the sheet-like structure (18) and fixes the layers (20, 22) of the sheet-like structure, and
e. spacing the stamps (80, 82) on both sides apart from the sheet-like structure (18) after the bearing structure (24) has cured.

15. Method for producing a protective cap (10) according to one of Claims 3 to 7, or a venting insert according to either of Claims 9 and 10, comprising the following steps:
a. providing a main body (8) of a vented protective cap or of a venting insert, which main body has a venting aperture which is surrounded by a bearing structure (26) having a ring-shaped fastening surface (28), and
b. placing a first layer (20) of the sheet-like structure and a second layer (22) of the sheet-like structure such that the boundary region (20A) of the first layer (20) comes into contact with the ring-shaped fastening surface (28) and the boundary region (22A) of the second layer (22) comes into contact with the boundary region (20A) of the first layer (20), and
c. effecting thermal joining-together of the boundary regions (20A, 22A) of the first layer (20) and second layer (22) by means of a heated stamp (86) after insertion of the second layer (22).

16. Method according to Claim 15, having the following feature:
a. the boundary region (20A) of the first layer is, by means of a heated stamp (86), already thermally bonded to the ring-shaped fastening surface (28) after the placement of the first layer (20) and before the placement of the second layer (22).

## Revendications

1. Capuchon de protection ventilé (10) pour un distributeur de liquide (100) destiné à distribuer un liquide pharmaceutique, présentant les caractéristiques suivantes :
a. le capuchon de protection (10) présente une paroi (12) de capuchon qui entoure un espace intérieur (14) de capuchon, et
b. la paroi (12) de capuchon présente un passage de ventilation (16) qui est recouvert par une structure de surface (18) flexible, et
c. une zone de bordure (18A) de la structure de surface (18) est fixée sur le pourtour d'un bord du passage de ventilation (16), et
d. le capuchon de protection (10) est apte à être placé sur une unité principale (102) du distributeur de liquide (100) et protège une ouverture de décharge (108) de l'unité principale (102) à l'état mis en place, **caractérisé par** la caractéristique suivante :
e. la structure de surface (18) se compose d'au moins deux couches perméables à l'air (20, 22) qui s'étendent de façon directement adjacente l'une à l'autre, mais qui ne sont pas fermement reliées entre elles au moins dans une zone centrale (18B) de la structure de surface (18), et
f. la structure de surface (18) recouvre le passage de ventilation (16) et garantit ainsi qu'un échange d'air entre un espace intérieur (14) du capuchon et un environnement n'est possible qu'à travers la structure de surface (18) lorsque le capuchon de protection (10) est mis en place.

2. Capuchon de protection ventilé (10) selon la revendication 1 avec la caractéristique supplémentaire suivante :
a. les deux couches (20, 22) de la structure de surface (18) sont maintenues sur le bord du passage de ventilation (16) par une structure porteuse commune (24) moulée par injection, qui dépasse de la zone de bordure (18A) de la structure de surface (18) d'un côté ou d'un seul tenant des deux côtés de la structure de surface.

3. Capuchon de protection ventilé (10) selon la revendication 1, ayant les caractéristiques supplémentaires suivantes :
a. une structure de support (26) avec une surface de fixation annulaire (28) est prévue sur le bord du passage de ventilation (16), et
b. une première couche (20) de la structure de surface est fixée thermiquement à la surface de fixation (28) dans une zone de bordure (20A), et
c. une deuxième couche (22) de la structure de surface (18) est fixée thermiquement dans une zone de bordure (22A) à la zone de bordure (20A) de la première couche (20).

4. Capuchon de protection ventilé (10) selon l'une des revendications précédentes ayant la caractéristique supplémentaire suivante :
a. le capuchon de protection (10) présente un élément principal (30) qui forme au moins en majeure partie une paroi d'enveloppe de la paroi (12) de capuchon et sur lequel la structure porteuse (24 ; 26) est prévue d'un seul tenant.

5. Capuchon de protection ventilé selon l'une des revendications 1 à 3, ayant les caractéristiques supplémentaires suivantes :
a. le capuchon de protection (10) présente un élément principal (32) qui forme au moins en majeure partie une paroi d'enveloppe de la paroi (12) de capuchon et qui présente une ouverture pour recevoir un insert de ventilation (34), et
b. le capuchon de protection (10) présente un insert de ventilation (34) inséré de manière étanche dans l'ouverture, qui comprend la structure porteuse (24 ; 26) environnante et la structure de surface (18) maintenue et entourée par celle-ci.

6. Capuchon de protection ventilé (10) selon l'une des revendications précédentes avec au moins l'une des caractéristiques supplémentaires suivantes :
a. au moins une couche de la structure de surface (18) est formée par un filtre stérile (21A, 21B) qui présente de préférence un seuil de coupure de 1 µm au maximum, en particulier de préférence un seuil de coupure de 0,5 µm au maximum ou de 0,2 µm au maximum, et/ou
b. au moins une couche de la structure de surface (18) est une couche absorbante (21C) qui est prévue pour recevoir une goutte résiduelle provenant d'une ouverture de distribution du distributeur, celle-ci représentant de préférence la première couche de la structure de surface (18) en partant de l'espace intérieur (14) de capuchon, et/ou
c. au moins une couche de la structure de surface (18) est conçue comme un filtre en profondeur (21B), et/ou
d. au moins une couche de la structure de surface (18) est conçue comme un filtre à membrane (21A), et/ou
e. au moins une couche de la structure de surface (18) présente une surface hydrophile, et/ou
f. au moins une couche de la structure de surface (18) présente une surface hydrophobe, et/ou
g. au moins une couche de la structure de surface (18) est conçue comme une couche antibactérienne (21C) et présente un ingrédient bactéricide, et/ou
h. au moins une couche de la structure de surface (18) est conçue comme une couche de support (21D), en particulier une couche la plus extérieure, et/ou
i. au moins une couche extérieure est conçue comme une couche de protection visuelle (21E) et présente une couleur uniforme avec la structure de support environnante, et/ou
j. le capuchon de protection (10) présente une structure de protection pour protéger la structure de surface, la structure de protection (29) étant réalisée sous forme de composant séparé de la structure porteuse (24) et étant reliée à la structure porteuse (24) par adhérence ou par complémentarité de forme.

7. insert de ventilation (34) pour un canal de ventilation destiné à être utilisé dans un capuchon de protection (10) selon la revendication 5 et à protéger un orifice de distribution (108) d'une unité principale (102) d'un distributeur de liquide (100) lorsque le capuchon de protection (108) est mis en place, ayant les caractéristiques suivantes :
a. l'insert de ventilation (34) présente un passage de ventilation (16) qui est entouré par une structure de support (24 ; 26), et
b. le passage de ventilation (16) est recouvert d'une structure de surface (18) flexible, et
c. une zone de bordure (18A) de la structure de surface (18) est fixée sur la structure porteuse (24 ; 26) sur le pourtour,
**caractérisé par** la caractéristique supplémentaire suivante :
d. la structure de surface (18) est constituée d'au moins deux couches (20, 22) perméables à l'air, qui s'étendent de façon directement adjacente l'une à l'autre, mais qui ne sont pas reliées de manière fixe l'une à l'autre au moins dans une zone centrale (18B) de la structure de surface (18), et
e. la structure de surface (18) recouvre le passage de ventilation (16) et garantit ainsi qu'un échange d'air entre un espace intérieur (14) de capuchon et un environnement n'est possible qu'à travers la structure de surface (18) lorsque le capuchon de protection (10) est mis en place.

8. insert de ventilation selon la revendication 7 présentant la caractéristique supplémentaire suivante :
a. les deux couches (20, 22) de la structure de surface (18) sont maintenues sur le bord du passage de ventilation (16) par une structure porteuse commune (24) moulée par injection, qui dépasse de la zone de bordure (18A) de la structure de surface (18) d'un côté ou d'un seul tenant des deux côtés de la structure de surface.

9. insert de ventilation selon la revendication 7 ayant les caractéristiques supplémentaires suivantes :
a. une structure de support (26) avec une surface de fixation annulaire (28) est prévue sur le bord du passage de ventilation (16), et
b. une première couche (20) de la structure de surface (18) est reliée thermiquement à la surface de fixation (28) dans une zone de bordure (20A), et
c. une deuxième couche (22) de la structure de surface (18) est reliée thermiquement, dans une zone de bordure (22A), à la zone de bordure (20A) de la première couche (20).

10. insert de ventilation selon l'une des revendications 7 à 9, ayant l'une des caractéristiques suivantes :
a. l'insert de ventilation (34) présente une surface d'enveloppe effilée pour un montage simplifié dans le canal de ventilation, ou
b. l'insert de ventilation (34) est fixé dans le canal de ventilation au moyen d'un assemblage par encliquetage, ou
c. l'insert de ventilation (34) est fixé dans le canal de ventilation au moyen d'une connexion filetée, ou
d. l'insert de ventilation (34) est fixé dans le canal de ventilation au moyen d'un assemblage par collage ou soudage.

11. Distributeur de liquide (100) pour liquides pharmaceutiques, ayant les caractéristiques suivantes :
a. le distributeur de liquide (100) dispose d'une unité principale (102) avec un réservoir de liquide (104), un dispositif de transport (106) et une ouverture de décharge (108) pour distribuer le liquide, et
b. le distributeur de liquide (100) dispose d'un capuchon de protection (10) qui est apte à être placé sur l'unité principale (102) et qui protège l'orifice de distribution (108) lorsqu'il est mis en place,
**caractérisé par** l'une des caractéristiques suivantes :
c. le capuchon de protection (10) est conçu selon l'une des revendications 1 à 6 et/ou
d. le capuchon de protection (10) comprend un insert de ventilation (34) selon les revendications 7 à 10.

12. Distributeur de liquide (100) selon la revendication 11, présentant les caractéristiques supplémentaires suivantes :
a. au moins une couche (22) de la structure de surface (18) est une couche absorbante ou antibactérienne (22), cette couche étant disposée directement au-dessus de l'ouverture de distribution (108) lorsque le capuchon de protection (10) est en place, de sorte qu'une goutte résiduelle de celle-ci restant au niveau de l'ouverture de distribution est apte à être recueillie et/ou décontaminée.

13. Distributeur de liquide (100) selon la revendication 11 ou la revendication 12 avec au moins une des caractéristiques supplémentaires suivantes :
a. le distributeur de liquide (100) est conçu sous la forme d'un distributeur de gouttes et présente une géométrie de formation de gouttes (110) entourant l'ouverture de distribution (108), et/ou
b. le distributeur de liquide (100) est rempli d'un liquide pharmaceutique, et/ou
c. le distributeur de liquide (100) présente un dispositif de pompage (106) pour le transport du liquide du réservoir de liquide (104) vers l'ouverture de distribution (108), et/ou
d. le distributeur de liquide (100) présente un flacon pressable (112) qui est apte à être comprimé dans le but de transporter le liquide du réservoir de liquide (104) vers l'ouverture de distribution (108), et/ou
e. le distributeur de liquide (100) présente un accumulateur de pression, et/ou
f. le distributeur de liquide (100) est conçu sous la forme d'un distributeur à pulvérisation, au moyen duquel le liquide est distribué sous forme pulvérisée.

14. Procédé de fabrication d'un capuchon de protection (10) selon l'une des revendications 2 et 4 à 7 ou d'un insert de ventilation selon l'une des revendications 8 ou 10, comprenant les étapes suivantes :
a. au moins une première couche d'un premier matériau plat et une deuxième couche d'un deuxième matériau plat sont amenées en continu sous forme de bandes plates (220, 222), et
b. dans une zone de chevauchement des bandes, les au moins deux couches sont comprimées par un poinçon supérieur (80) et un poinçon inférieur (82) et une coupe est effectuée avec un contour de découpe (84) en entourant les poinçons (80, 82), par laquelle les deux couches (20, 22) d'une structure de surface (18) prévue pour le capuchon de protection (10) ou l'insert de ventilation (34) sont séparées des bandes plates (220, 222), et
c. la structure de surface multicouche (18) est insérée dans une cavité de moulage par injection (90) à l'état fixé par les poinçons (80, 82), et
d. une matière plastique (92) est injectée dans la cavité de moulage par injection (90), qui forme une structure porteuse commune (24) qui dépasse de la zone de bordure (18A) de la structure de surface (18), d'un côté ou d'un seul tenant des deux côtés de la structure de surface (18), et qui fixe les couches (20, 22) de la structure de surface, et
e. les poinçons (80, 82) situés des deux côtés sont espacés de la structure de surface (18) après le durcissement de la structure porteuse (24).

15. Procédé de fabrication d'un capuchon de protection (10) selon l'une des revendications 3 à 7 ou d'un insert de ventilation selon l'une des revendications 9 ou 10, comprenant les étapes suivantes :
a. il est fourni un corps de base (8) d'un capuchon de protection ventilé ou d'un insert de ventilation qui dispose d'une ouverture de ventilation qui est entourée d'une structure de support (26) avec une surface de fixation annulaire (28), et
b. une première couche (20) de la structure de surface et une deuxième couche (22) de la structure de surface sont placées de telle sorte que la zone de bordure (20A) de la première couche (20) vient en contact avec la surface de fixation annulaire (28) et que la zone de bordure (22A) de la deuxième couche (22) vient en contact avec la zone de bordure (20A) de la première couche (20), et
c. on réalise, au moyen d'un poinçon chauffé (86), après insertion de la deuxième couche (22), un assemblage thermique des zones de bordure (20A, 22A) de la première couche (20) et de la deuxième couche (22) entre elles.

16. Procédé selon la revendication 15 ayant la caractéristique suivante :
a. après la mise en place de la première couche (20) et avant la mise en place de la deuxième couche (22), la zone de bordure (20A) de la première couche est reliée thermiquement à la surface de fixation annulaire (28) au moyen d'un poinçon chauffé (86).
